Europäisches Patentamt

European Patent Office

Office européen des brevets

(19)

(11) Publication number:

**0 331 505**
**A1**

(12)

# EUROPEAN PATENT APPLICATION

(21) Application number: **89302121.2**

(22) Date of filing: **03.03.89**

(51) Int. Cl.⁴: **A 61 K 9/50**

(30) Priority: **07.05.88 JP 110792/88**
**04.03.88 JP 52249/88**

(43) Date of publication of application:
**06.09.89 Bulletin 89/36**

(84) Designated Contracting States:
**AT BE CH DE ES FR GB GR IT LI LU NL SE**

(71) Applicant: **TAKEDA CHEMICAL INDUSTRIES, LTD.**
**3-6, Doshomachi 2-chome Chuo-ku**
**Osaka 541 (JP)**

(72) Inventor: **Uda, Yoshiaki**
**2-501, 3 Nikawadanchi**
**Takarazuka Hyogo 665 (JP)**

**Iga, Katsumi**
**A-808, 1 Satsukigaokanishi**
**Suita Osaka 563 (JP)**

**Hoshino, Tetsuo**
**13-6, Shin-kofudai, Toyono-cho**
**Toyono-gun Osaka 563-01 (JP)**

(74) Representative: **Lewin, John Harvey et al**
**ELKINGTON AND FIFE Beacon House 113 Kingsway**
**London WC2B 6PP (GB)**

(54) A method for purification of liposome composition.

(57) The drug-entrapping liposome composition can be purified advantageously in the practical use by subjecting an aqueous fluid containing drug-entrapping liposomes and the unentrapped drug to dialysis through hollow fibers, thereby separating and removing the unentrapped drug into the fluid flowing outside the hollow fibers.

**Description**

## A Method for Purification of Liposome Composition

This invention relates to the method for purification of drug-entrapping liposome compositions.

After preparation of drug-enrapping liposomes from a phospholipid and a solution of a drug, a process is generally necessary to separate the unentrapped free drug from the drug-entrapping liposomes. The techniques known for the separation include dialysis in cellophane bags [Saibo Kogaku (Cell Technology), 2, 1136 (1983)], ultracentrifugtion [Biochemistry, 17, 1792 (1978)], and gel filtration [Biochemistry, 8, 344 (1969)].

As described above, there have been several methods for separation-removal of the free drug unentrapped in liposomes, though they are not suitable for industrial application. For example, the method using cellophane bags has drawbacks such as troublesome dispensation in aliquots of the liposome suspension to be treated, a long time required for the treatment, inconsistent quality, difficulty in aseptic producton, etc. Separation based on ultracentrifugation is achieved by several repetitions of the process which comprises dispensing in aliquots of the liposome suspension in the ultracentrifuge tubes, ultracentrifugation, discarding the supernatant, and collecting the deposit, and therefore the operation may be also troublesome and aseptic production may be difficult. In addition some types of liposome [for example SUV (Small unilamellar vesicle) REV (reverse-phase evaporation vesicle], are difficult to be recovered completely because they will not sediment well and be discarded together with the supernatant. Therefore the method is applicable only to liposomes with relatively large diameters, and some types of liposome may form aggregate on re-dispersion because they have aggregated during sedimentation by centrifugation.

Separation based on gel filtration is achieved by allowing to flow the liposome suspension through a column packed with an adsorbent to adsorb the unentrapped drug. This method has drawbacks that a long time is required for the treatment because of the relatively slow flow through the column, that a concentration process is required afterwards because the suspension has been diluted with the eluent for the column, that a pretreatment of the column with empty liposomes is required because the phospholipid may be adsorbed onto the adsorbent, that mechanical blocking at the inlet of the column may occur occasionally, that regeneration of the adsorbent (removal of the adsorbed drug by liberation) is required for re-use of the adsorbent, that aseptic operation (sterilization of the adsorbent before use) is difficult, etc.

As described above, the method for separation and removal of the unentrapped free drug in the course of production of drug-entrapping liposome compositions has not been established yet. For industrial application, the method should satisfy as far as possible the requirements that a large amount can be treated in a short time, that aseptic operation is easy, that final products can be obtained within a day by a through process by connecting with the processes for production of liposome composition, etc.

As the result of the inventors' researches under the circumstances described above, they found out that a hollow fiber-type dialyzer can be used for sepration and removal of the unentrapped drug in the course of production of liposome compositions, and have completed this invention.

Namely, this invention is the method for purifcation of liposome compositions characterized in that an aqueous fluid containing drug-entrapping liposomes and the unentrapped drug is dialyzed through the hollow fibers, and the unentrapped drug is separated and removed into the fluid flowing outside the fiber.

"The aqueus fluid containing drug-entrapping liposomes and the unentrapped drug" in this invention is not subject to any restriction as far as it is a system wherein drug-entrapping liposomes are suspended in water and at the same time a drug is contained in the water. Generally it is fluid obtained after production of drug-entrapping liposomes according to the per se known method using a phospholipd and a drug solution, and the aqueous fluid wherein liposomes are suspended and the unentrapped drug is contained is the main subject of this invention. In this specification the aqueous fluid described above is sometimes called simply as "the liposome suspension". In the production of drug-entrapping lippsome compositions according to the methods known so far, occurrence of unentrapped drug is inevitable. It is generally desirable that the unentrapped drug is removed prior to use of a liposome composition for DDS (Drug Delivery System) etc. The liposomes to be included in this invention are of any type, i.e. any of SUV, MLV (multilamellar vesicle), LUV (large unilamellar vesicle), REV, or SPLV (stable plurilamellar vesicle).

The hollow fiber dialyzer used in this invention is a device which is composed generally of several thousands of hollow fibers made of semipermeable membrane material and in which the solution to be dialyzed is allowed to flow outside the fibers. Such a device is exemplified by a dialysis device constructed similarly to the known hollow fiberhemodialyzer. The material of hollow fibers may be selected among natural polymer membranes and the processed products thereof, regenerated polymer membranes, and synthetic polymer membranes, on the basis that the material should not react with or adsorb phospholipid, the material of liposomes.

In the concrete, natural polymer membranes and the processed products thereof include cellophane and crosslinking collagen, regenerated polymer membranes include various cellulose membranes such as cellulose acetate (for example, oxidation; 50% or more), cellulose triacetate, cellulose acetate butyrate, nitrocellulose, and cupro ammonium rayon membranes.

Synthetic polymer membranes that are being studied include polyvinyl alcohol, polyvinylpyrrolidone methylene-bis (4-phenylisocyanate)(MDI), polyethyleneglycol MDI, polybisphenol carbonate-polyethylene oxide copolymer, polyion complex, polypropylene, polyvinyl acetate, poly-sulfone carbonate, polyethylene, polyacrilonitrile, and polymethylmethacrylate membranes. Among these, polyvinyl alcohol MDI, cross-linking collagen, polybisphenol carbonate polyethylene oxide, polyvinyl acetate, polysulfone carbonate, polyacrylnitrile, polymethylmethacrylate membranes are desirably used in this invention.

Permeability of the hollow fiber membranes, when expressed in the half-value time of the rate of dialysis of urea, is desirably equivalent to or smaller than that of cellophane; for example, membranes with the permeability of about 0.1 to 1, taking the half-value time of cellophane as 1, are used advantageously.

The selection of a hollow fiber with a permeability in this invention is dependent also on the molecular size of the drug used, and this point is explained in the following.

The drugs employable for production of liposome compositions are water-soluble and usable in micro-capsules. The solubility in water should be such that the logarithms of the partition coefficient in the octanol-water system is 10 or less. Hollow fibers of such a pore size that allows to permeate the drug but not the liposomes are selected, and the permeability of the drug is dependent on the pore size of the fiber and the molecular size of the drug. The pore size of the fiber is a measure of permeability; the pore size of 6 Å correspnds to the molecular size of creatinine (molecular weight 113), 15 Å to that of vitamin $B_{12}$ (mol. wt. 1,350), 24 Å to that of inulin (mol. wt. 5,200), 32 Å to that of cyto-chrome C (mol. wt. 24,000), 46 Å to that of β-immunoglobulin (mol. wt. 38,000), and 55 Å to that of albumin (mol. wt. 60,000). The pore size of cupro ammonium rayon often used as a hollow fiber membrane for the hollow fiber dialyzer is about 20 Å. Drugs of molecular weight of 3,000 or less can be dialyzed efficiently through a hollw fiber membrane of a pore size of about 20 Å.

The desirable drugs employable for this invention are those with molecular weight more than 100 and less than about 60,000. The employable drugs for this invention are classified by the molecular weight in the following.

(1) drugs with molecular weight more than 100 and less than 1000

(Example) Metal complexes such as cisplatin (CDDP), carboplatin, iproplatin, tetraplatin, etc., antitumor antibiotics such as mitomycin, adriamycin, ansamitocin, actinomycin, and the derivatives thereof (e.g. 9-thiomeitancin), bleomycin, Ara-C, daunomycin, etc., metabolic antagonists such as 5-FU, methotrexate, TAC-788 [isobutyl 5-fluoro-6-(E)-furfurylideneaminoxy-1,2,3,4,5,6-hexahydro-2, ,4-dioxopyrimidine-5-carboxylate, Japanese Unexamined Patent Publication No. 59-13780], etc., alkylating agents such as BCNU, CCNU, etc.; antitumor agents such as melphalan, mitoxanthoron, etc., aminoglycoside antibiotics such as gentamcyn, streptomycin, canamycin, dibekacin, paromomycin, kanendomycin, lipidomycin, tobramycin, amikacin, fradiomycin, sisomicin, etc, β-lactam antibiotics including penicillins such as sulbenicillin, mecillinam, carbenicillin, piperacillin, ticarcillin, etc., thienamycins; cephalosporins such as cefotiam, cefsulodin, cefmenoxime, cefmetazole, cefazolin, cefotaxime, cefoperazone, ceftizoxime, mexalactam, etc., TRH drugs [TRH, TRH analogues (e.g. L-N-(2-oxopiperazine-6-carbonyl)-L-histidyl-L-thiazolidine-4-carboxamide, L2-oxooxazoline-4-carbonyl-L-histidyl-L-prolinamide, L-trans-5-methyl-2-oxooxazolidine-4-carbonyl-L-histidyl-L--prolinamide, L-2-oxothiazolidine-4-carbonyl-L-histidyl-L-prolinamide, γ-butyllactone-carbonyl-L-histidyl-L-prolinamide, 2-ketopiperidine-6-carbonyl-L-histidyl-L-prolinamide, 3-oxoperhydro-1,4-thiazine-5-carbonyl-L-histidyl-L-prolinamide), the compounds obtained by the substitution of the hydrogen atom of the amide group in these TRH and TRH analogues with methyl, ethyl, n-propyl, n-butyl, n-hexyl, n-amyl, or β-phenethyl group, and their acetates, tartarates, etc.], peptides such as enkephalin, etc., anti-inflammatory agents, analgesics, and anti-pyretics such as aspirin, diflunisal, indomethacin, diclofenac, fenbufen, sulindac, acemetacin, ibuprofen, naproxen, ketoprofen, flurbiprofen, fenoprofen, thiaprofen, pronoprofen, mefenamic acid, tolfenamic acid, flufenamic acid, phenylbutazone, oxyphenbutazone, pyroxicam, mepirizole, emorfazone, etc., and various prostaglandins and the derivatives thereof.

(2) Drugs with molecular weight more than 1000 and less than 2000

(Examples) peptide antibiotics such as gramicidin D, bacitracin, etc.

(3) Drugs with molecular weight more than 2000 and less than 5000

(Examples) peptides such as ACTH, polymyxin B, colistin, etc.

(4) Drugs with molecular weight more than 5000 and less than 10000

(Examples) polysaccharides such as heparin, lentinan, zymosan, PS-K, etc., peptide hormones such as insulin, growth hormone, etc.

(5) Drugs with molecular weight more than 10000 and less than 20000

(Examples) limphokines such as interferon (α, β, γ), interleukin 2, TNF, etc.

(6) Drugs with molecular weight more than 20000 and less than 60000

(Example) SOD

The hollow fibers used are desirably those with the inner diameter of the hollow ranging from about 150 to 250 μm and with the membrane aobut 10 to 20 μm in thickness. For hollow fiber dialyzers, a thick fiber with a large surface area may be used, but it is advantageous for easier treatment in a large scale that two or more hollow fibers each of which has a small surface area are connected in parallel or in series. It is more desirable to use a bundle of many hollow fibers; for example, a bundle of 9900 hollow fibers each of which has the inner diameter of 200 μm and the effective length of 23.5 cm provides a dialyzer having the dialyzing area of 1.5 $m^2$, and a dialyzer of a surface area of such a magnitude is practically used. The dialysis area may be enlarged by connecting some bundles of the hollow fibers in series or in parallel.

In the dialysis of this invention, the drug which has permeated through the membrane is removed from the system by allowing a fluid to flow outside the hollow fibers. Water may be used as the fluid flowing outside the fibers, but use of a fluid appropriate for the dosage form of the liposome preparation is practically preferable. That is, aqueous solutions of various substances which are physiologically accetable to warm-blooded animals are used, such as, in the concrete, aqueous solutions of salts (e.g. edible salt), sugars (e.g. glucose, mannitol, sorbitol), amino acids (e.g. glycine, asparatic acid, glutamic acid). Among these, physiological saline is the suspension medium that is used most commonly for intravenous administration of various drugs, and can be used advantageously also in this invention.

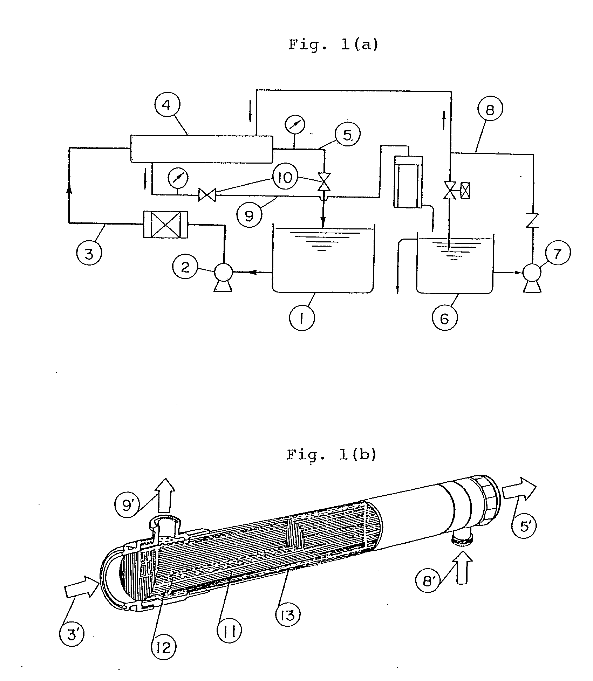

In the device illustrated in Fig. 1 (a), a liposome suspension is poured into the reservoir (1) and allowed to flow by the pump (2) into the hollow fiber dialyzer (4) connected through the pipe (3) with the reservoir. The structure of the dialyzer (4) is shown in Fig. 1 (b); the hollow fibers (11) are bundles at the jointing and placed in the case (13) which has an inlet (8') and an outlet (9') for the dialyzing fluid and the dialyzing fluid flows outside the hollow fibers in this case. The liposome suspension is sent to the inlet (3') of the dialyzer (4), allowed to flow into the hollow fibers, and brought back from the outlet (5') through the pipe (5) into the reservoir (1). The dialyzing fluid placed in the reservoir (6) in Fig. 1 (a) is allowed to flow by the pump (7) through the pipe (8), the inlet (8'), the outlet (9'), and the pipe (9) for the dialyzing fluid of the hollow fiber dialyzer described above to the outside of the dialyzer. The dialyzing fluid can be recycled if necessary after removal by adsorption of the drug onto active carbon or ion exchange resin. Thus the free drug in the liposome suspension is gradually removed from the system by being dissolved in the dialyzing fluid through the membrane of the hollow fibers, and a purified liposome composition can be recovered in the reservoir (1).

In this invention, the free drug may be removed efficiently when dialysis is performed while the pressure inside the hollow fibers is loaded to be higher than that of the dialyzing fluid. The pressure difference is generally controlled to be 300 mmHg or less, preferably about 50 mmHg or less, more desirably 10 to 50 mmHg. When the difference is 50 mmHg or more, also water permeates much, which results in concentration of the liposome suspension and therefore it is possible to remove the unentrapped free drug and at the same time to concentrate the suspension. However concentration under an excessively different pressure is undesirable because the drug entrapped in liposomes may leak out. The pressure difference is adjusted, for excample, with the pressure difference-controlling valves (10) equipped in the liposome suspension-sending pipe (5) and the dialyzing fluid-sending pipe (9) in the device shown in Fig. 1 (a). Particularly for removal of a drug with high molecular weight (for example, molecular weight of 10000 to 60000) of which dispersion-dialysis rate is low, a hollow fiber membrane dialysis is performed efficiently by applying a pressure to the fluid inside the hollow fibers; therefore a membrane thick enough to resist the pressure is desirable for this purpose.

The liposome suspension and the dialyzing fluid are generally allowed to flow in countercurrent, but may be allowed to flow in parallel in some cases. Two or more devices (4) for the hollow fiber dialysis may be connected in series or in parallel to be suited for treatment in a large scale.

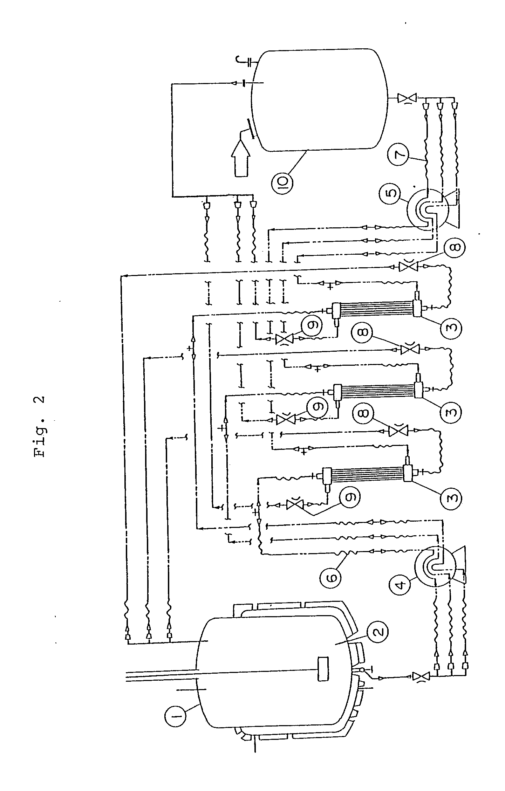

More practically the operation efficiency can be elevated by performing the production of a liposome composition and separation-removal of the unentrapped drug in a through process in a closed system, as shown in Fig. 2, which connects directly the apparatus for production of the liposome composition and the hollow fiber dialyzer such as that illustrated in Fig. 1 (b). In the process the purified liposome composition can be produced aseptically by previous treatment such as high pressure steam sterilization of the hollow fiber dialyzer.

According to the invention, from an aqueous fluid containing drug-entrapping liposomes and the unentrapped drug, the unentrapped drug can be separated and removed practically advantageously, and the method of this invention is very useful for purification of liposome compositions. That is, as compared to the conventional methods based on dialysis in a bag, ultracentrifugation, or gel filtration, the method of this invention is very suitable for practically use because it permits a large scale treatment in a short time, sterilization of the dialyazer, and treatment in a closed system by connecting the dialyzer directly to the liposome-producing apparatus. The method of this invention is excellent also because it does not cause any damage to the liposomes or leaking of the entrapped drug.

Brief Description of the Drawings

Fig. 1(a) is an example of the device used in this invention. In this figure, (1) is the reservoir of liposome suspension, (2) liposome suspension sending pump, (3) and (5) liposome suspension sending pipes, (4) hollow fiber dialyzer, (6) reservoir of dialyzing fluid, (7) dialyzing fluid sending pump, (8) and (9) dialyzing fluid sending pipes, and (10) pressure difference controlling valve.

Fig. 1(b) is an enlarged figure of the hollow fiber dialyzer (4) in Fig. 1(a). (11) is the hollow fiber membrane (hollow fibers), (12) jointing of the bundle of hollow fibers, (13) case, (3') liposome suspension inlet (before dialysis), (5') liposome suspension outlet (after dialysis), (8') dialyzing fluid inlet, and (9') dialyzing fluid outlet.

Fig. 2 is an example of the device used for production of drug-entrapping liposomes and separation of the unentrapped drug in an aseptic through process by connecting the liposome-producing apparatus directly to the hollow fiber dialyzer. In this figure, (1) is liposome-producing apparuts (vacuum emulsator), (2) liposome suspension, (3) hollow fiber dialyzer, (4) liposome suspension-sending pump, (5) dialyzing fluid-sending pump, (6) liposome suspension-sending pipe, (7) dialyzing fluid-sending pipe, (8) valve controlling the pressure inside the hollow fibers, (9) valve controlling the pressure of dialyzing fluid, and (10) reservoir of dialyzing fluid.

4

The following Experimental Examples and Examples will explain the present invention in more detail, but do not restrict the invention at all.

Experimental Example 1

(1) Production of REV liposomes

In a 1 ℓ round-bottomed flask, 300 ml of 0.5 mM 6-CF (6-carboxyfluorescein) in physiological saline and 6 g of a phospholipid [dipalmitoylphosphatidyl choline (DPPC): distearylphosphatidyl choline (DSPC) = 9:1] dissolved in 300 ml of a mixture of chloroform : diisopropyl ether (1:1) were mixed and emulsified by ultrasonic irradiation for 20 minutes in a bath-type ultrasonicator (60 W). The emulsion was subjected to gradual evaporation of the organic solvent on a warm water bath at 55°C by a vacuum rotary evaporator, to prepare a suspension of 6-CF-entrapping REV liposomes. In this suspension 6-CF-entrapping in liposomes was also included.

(2) Dialysis

Reference dialysis: In a cellophane bag (inner diameter; 2 cm, length; 1 m, effective surface area; 0.08 m², Spectrapore, manufactured by SPECTRUM MEDICAL INDUSTRIES, INC.), 300 ml of the liposome suspension was packed, the bag was immersed in 10 ℓ of physiological saline, and dialysis was performed with stirring at room temperature.

Dialysis according to this invention: Three hundred ml of the liposome suspension was dialyzed in a dialyzer using hollow fibers made of cupro ammonium rayon (mean inner diameter of the hollow; 200μm, effective surface area; 0.8 m², Type TAF08W, manufactured by Thermo Co.) at the flow rate of the liposome suspension of 150 ml/min., the flow rate of the dialyzing fluid (physiological saline) of 500 ml/min., and the difference between the pressure of the suspension and that of the dialyzing fluid of 10 mmHg.

(3) Measurement of the efficiency of separation-removal by dialysis

The efficiency of separation-removal of the unentrapped free 6-CF was expressed in the percentage of the content of free 6-CF in the liposome susspensin before the separation-removal relative to the content after the separation-removal (remaining percentage).

(4) Determination of free 6-CF

The liposome suspension, 0.1 ml, was placed in a Centrisart (Centrisart 1, SM13249, manufactured in West Germany), to which 1 ml of physiological saline was added, and centrifuged (2000 rpm, 5 minute). A fixed amount of the supernatant containing only free 6-CF was diluted 100 times with distilled water and put into a quarts cell of 1 cm light-path length. The intensity of the fluorescence was measured with a fluorometer (excitation wave length; 494 nm, fluorescence wave length; 515 nm), and the amount of free 6-CF was estimated from the calibration curve. The calibration curve was prepared by measuring the intensity of fluorescence of the standard solutions of 6-CF which had been prepared separately.

(5) Results

Table 1 shows the relation between the treatment time and the remaining percentage of 6-CF in the reference dialysis and in the dialysis according to the present invention.

Table 1

| time (hr) | remaining percentage of 6-CF (%) | | | | | | | | |
|---|---|---|---|---|---|---|---|---|---|
| | 0 | 0.08 | 0.25 | 0.5 | 0.66 | 6 | 24 | 48 | 72 |
| reference dialysis | 100 | – | – | – | – | 78 | 27 | 6 | <0.8 |
| this invention | 100 | 25 | 3 | <1.0 | <0.1 | – | – | – | – |

As evident from the results shown in Table 1, the remaining percentage of free 6-CF unentrapped in liposomes became below 0.8% only after 72 hours (3 days) of dialysis by the reference method, whereas the remaining percentage of free 6-CF became below 0.1% in 40 minutes of dialysis according to the method of this invention; thus the method of this invention can separate and remove the free drug efficiently in a very short time.

When hollow fibers with the effective surface area of 1.5 m$^2$ were used in the dialysis of this invention described above, the remaining percentage of 6-CF became below 0.1 % only in 20 minutes of dialysis, thus the time of treatment being shortened further.

Experimental Example 2

The suspension of 6-CF-entrapping liposomes prepared in the same way as in the Experimental Example 1 was used to compare the efficiency of separation of the unentrapped 6-CF by ultracentrifugation (12000 x g, 30 minutes) and that by the method of this invention.

By ultracentrifugation liposomes with smaller particle size were not sedimented well, floating in the supernatant, and flowed out by decantation, which resulted in a large loss of liposomes. The efficiency of separation-removal expressed in the remaining percentage of 6-CF was as large as 6.3 % after a run of ultracentrifugation, and became below 1% only after 3 repetitions of washing and ultracentrifugation, which required as long as about 90 minutes, making a large-scale treatment difficult. In contrast to this method, the method of this invention could be applied to various liposomes and permitted a large-scale treatment in a very short time with little loss of liposomes.

Experimental Example 3

(1) Preparation of cisplatin (CDDP)-entrapping REV

CDDP-entrapping liposomes were suspended in the same way as in the production of REV in the Experimental Example 1 except that CDDP (300 mg) was used instead of 6-CF, and thus a fluid in which unentrapped CDDP was dissolved was obtained.

(2) Dialysis

Reference dialysis: Similarly to the Experimental Example 1, 300 ml of the CDDP-entrapping liposome suspension was packed in a cellophane bag and treated.

Dialysis according to the method of this invention: Under the same conditions as those in the Experimental Example 1, 300 ml of the CDDP-entrapping liposome suspension was treated.

(3) Measurement of the efficiency of separation-removal by dialysis

The unentrapped free CDDP in the liposome suspension described above was determined according to the same method as described in the paragraph (3) of the Experimental Example 1, before and after the separation-removal, and the remaining percentage was determined.

(4) Determination of free CDDP

Free CDDP was determined in the same procedure as described in the paragraph (4) in the Experimental Example 1; CDDP-entrapping liposomes were separated from free CDDP in Centrisart (Centrisart 1, SM 13249, manufactured in West Germany), 0.1 ml of the supernatant containing only free CDDP was combined with 0.1 ml of an internal standard solution, and a fixed quantity of this solution was analyzed quantitatively by high performance liquid chromatography (HPLC).

The HPLC were conducted under the following conditions.

column: Hitachi Gel 3013N 4mm φ x 15 cm

column temperature: 50°C

eluent: 0.02 M $KH_2PO_4$-HCl (pH 3.0)

flow rate: 0.9 ml/min., detection: UV 210 nm

internal standard solution: aqueous solution of hypoxanthine (4 μg/ml)

The results are summarized in Table 2.

Table 2

| time (hr) | remaining percentage of CDDP (%) | | | | | | | | |
| | 0 | 0.08 | 0.25 | 0.33 | 0.5 | 6 | 24 | 48 | 72 |
|---|---|---|---|---|---|---|---|---|---|
| reference dialysis | 100 | – | – | – | – | 78 | 27 | 6 | <0.8 |
| this invention | 100 | 21 | 4.0 | 1.2 | <0.2 | – | – | – | – |

As evident from the results shown in Table 2 the remaining percentage of unentrapped free CDDP became 0.8% only after 72 hours (3 days) of dialysis by the reference method, whereas the remaining percentage became 0.2% in 30 minutes of dialysis according to the method of this invention; thus the method of this invention can separate and remove the free drug efficiently in a very short time.

Example 1

From 500 ml of a solution containing 1 mg/ml of cisplatin (CDDP) in physiological saline and 500 ml of a solution of 10 g of phospholipid (dipalmitoylphosphatidyl choline: distearylphosphatidyl choline = 9:1) in a mixture of chloroform: diisopropyl ether (1:1), CDDP-entrapping REV liposomes were produced according to the method described in the Experimental Example 1. The liposome suspension was dialyzed in a hollow fiber dialyzer (effective surface area; 1.5 m², inner diameter; 200 μm, membrane thickness; 20μm, cupro ammonium rayon, Type TAF-15W manufactured by Thermo Co.) by adjusting the flow rate of the liposome suspension circulating inside the hollow fibers to 150 ml/min., the flow rate of the dialyzing fluid (physiological saline) to 500 ml/min., and the pressure difference between the liposome suspension circulating and flowing out of the system and the dialyzing fluid to 10 mmHg. Dialysis under these conditions for 0.5 hours could separate and remove the unentrapped free CDDP. Thus a liposome composition was obtained which does not include any free CDDP with 20% entrapment of CDDP.

Example 2

Five hundred ml of a CDDP-entrapping REV liposome suspension prepared in the same way as in the Example 1 was dialyzed in a hollow fiber dialyzer (effective surface area; 1.5 m², cupro ammonium rayon, Type TAF-15W manufactured by Thermo Co.) by adjusting the pressure difference between the liposome suspension circulating and flowing out of the system and the dialyzing fluid to 70 mmHg by tightening the pressure difference-controll valve so as to elevate the pressure of the circulating liposome suspension. The treatment under these conditions for 0.3 hours provided a liposome preparation with 20% entrapment of CDDP in which the remaining percentage of the unentrapped free CDDP was less than 0.5% and the volume of the liposome dispersion had been reduced to 80% of the initial volume.

Example 3

One thousand ml of a CDDP-entrapping liposome suspension was prepared in the same way as in Example 1. The suspension was subjected to separation-removal of the unentrapped CDDP in a dialyzer composed of two hollow fiber dialyzers (effective surface area; 1.5 m², cupro ammonium rayon, Type TAF-15W manufactured by Thermo Co.) connected in parallel by adjusting the pressure difference between the liposome suspension and the dialyzing fluid to 10 mmHg for 0.5 hours, and a liposome composition was obtained which contained substantially no free CDDP with 20% entrapment of CDDP.

Example 4

Six hundred ml of a solution of 0.6 g of CDDP in physiological saline and 600 ml of a solution of 12 g of phospholipid (dipalmitoylphosphatidyl choline: distearylphosphatidyl choline = 9:1) in an organic solvent [chloroform: diisopropyl ether (1:1)] were emulsified in a vacuum emulsator (Agihomomixer M type, manufactured by Tokushu Kika Kogyo Co.) with a homomixer (revolution; 15000 rpm, 30 minutes), and the organic solvent was gradually evaporated off by stirring with a paddle mixer (80 rpm.) at 60°C under reduced pressure, to give CDDP-entrapping REV liposomes. The liposome suspension, 1150 ml, was dialyzed in a hollow fiber dialyzer (effective surface area; 1.5 m², cupro ammonium rayon, Type TAF-15W manufactured by Thermo Co.) by adjusting the rate of circulation and flowing out of the liposome suspension to 150 ml/min., the flow rate of physiological saline as the dialyzing fluid to 500 ml/min., and the pressure difference between the former and the latter to 10 mmHg, and thus a liposome preparation (1150 ml) with 20% entrapment of CDDP was obtained in 1.2 hours.

Example 5

The hollow fiber dialyzer the same as used in the Example 4 (effective surface area; 1.5 m², cupro ammonium rayon, Type TAF-15W manufactured by Thermo Co.) was preliminarily steam-sterilized unde elevated pressure. A vacuum emulsator (Agihomomixer M type, manufactured by Tokushu Kika Kogyo Co.), a liposome producing apparatus, was connected directly to the dialyzer, and the liposome suspension was allowed to circulate inside the hollow fibers in the closed system (Fig. 2) immediately after preparation of liposomes in the same method as in the Example 4, by using physiological saline as the dialyzing fluid, and by adjusting the flow rate to 500 ml/min. and the pressure difference between the liposome suspension and the dialyzing fluid to 10 mmHg. The unentrapped free CDDP was separated and removed under these conditions and a sterile liposome preparation was produced in a through process. After one hour of dialysis, a liposome suspension (1000 ml) with 20% entrapment of CDDP was obtained.

Example 6

One hundred ml of a solution of 1 g of egg yolk lecithin in chloroform was placed in a 500 ml eggplant-shaped flask and chloroform was gradually evaporated off by a vacuum rotary evaporator so that a thin film of phospholipid might be formed on the glass wall. To this was added 60 ml of a solution containing 1 mg/ml of CDDP in physiological saline, and stirred well so that CDDP might be entrapped, to produce MLV dispersed in the CDDP solution. Five batches of MLV prepared by this procedure were combined to make 300 ml, which was dialyzed in a hollow fiber dialyzer (cupro ammonium rayom, effective surface area; 0.8 m², Type TAF08, manufactured by Thermo Co.) at the circulating flow rate of liposome suspension of 150 ml/min., the flow rate of dialyzing fluid (physiological saline) of 500 ml/min., to remove the unentrapped free CDDP, and a liposome (300 ml) with 3% entrapment was obtained in 0.3 hours.

Example 7

In place of 1 mg/ml of CDDP used in Example 1, 200 mg/ml of 5-fluorouracil (5-FU) was used to prepare a 5-FU-entrapping liposome suspension according to the same procedure as in Example 1. The unentrapped free 5-FU was separated and removed by dialysis under the same conditions and a liposome suspension (300 ml) with 22% entrapment was obtained in 0.5 hours.

Example 8

According to the same procedure as in Example 1 except that 200 μg/ml of mitomycin C (MMC) was used in place of 1 mg/ml of CDDP in Example 1, and MMC-entrapping liposome composition was produced. The unentrapped free MMC was separated and removed by dialysis under the same conditions, and a liposome suspension (300 ml) with 24% entrapment was obtained in 0.5 hours.

Example 9

According to the same procedure as in Example 1 except that 500 μg/ml of Ara-C was used in place of 1 mg/ml of CDDP in Example 1, an Ara-C-entrapping liposome composition was produced. The unentrapped free Ara-C was separated and removed by dialysis under the same conditions, and a liposome suspension (300 ml) with 20% entrapment was obtained in 0.5 hours.

Example 10

According to the same procedure as in Example 1 except that 200 μg/ml of bis(chloroethyl)nitrosourea (BCNU) was used in place of 1 mg/ml of CDDP in Example 1, a BCNU-entrapping liposome composition was produced. The unentrapped free BCNU was separated and removed by dialysis under the same conditions, and a liposome suspension (300 ml) with 20% entrapment was obtained in 0.5 hours.

Example 11

18 g of DPPC and 2 g of DSPC were dissolved in 1000 ml of the 1:1 mixture of chloroform and isopropyl ether in a 2 1-beaker. To this solution was added 1000 ml of a solution of CDDP of 500 μg/ml in saline which had been prepared beforehand so that the osmotic pressure might be 1.9 times higher than that of physiological saline, mixed slightly, and emulsified in an emulsator (homomixer for 3 1, Tokushukika, Japan) for 60 minutes, to give a W/O emulsion. The emulsion thus obtained was subjected to evaporation of the organic solvent in the same homemixer at 60°C under reduced pressure, to give LUV. A portion (500 ml) of the resultant LUV was poured into an apparatus for dialysis (hollow fiber manufactured by Asahi Kasei, Japan: fiber length; about 25 cm, effective membrane area; 1.5 m², inner diameter; 200 μm, membrance thickness; 20 μm,) at the rate of about 150 ml/min. and physiological saline as the dialyzing fluid was allowed to flow at the rate of about 500 ml/min. so that the membrane pressure on the hollow fiber might become 0; thus free CDDP was removed by dialysis, and a liposome composition entrapping CDDP together with the hypertonic solution described above was obtained.

## Claims

1. A method for purification of liposome compositions, which comprises subjecting an aqueous fluid containing drug-entrapping liposomes and the unentrapped drug to dialysis through hollow fibers, and separating and removing the unentrapped drug into the fluid flowing outside the hollow fibers.

2. The method according to Claim 1, wherein the pressure of the aqueous fluid flowing inside the hollow fiber is loaded to be higher than that of the fluid flowing outside the fiber.

3. The method according to Claim 2, wherein the pressure difference is 300 mmHg or less.

4. The method according to Claim 1, wherein the hollow fiber is a cellulose membrane.

5. The method according to Claim 1, wherein the hollow fiber has a membrane with the permeability of about 0.1 to 1, and with the thickness of about 10 to 20 μm, and a hollow with the inner diameter of about 150 to 250 μm.

6. The method according to Claim 1, wherein the drug has a molecular weight more than about 100 and less than about 60,000.

7. The method according to Claim 1, wherein the drug is an antitumor agent having a molecular weight more than about 100 and less than 60,000.

8. The method according to Claim 1, wherein the water-solubility of the drugs is 10 or less as the logarithms of the partition coefficient in the octanol-water system.

Fig. 1(a)

Fig. 1(b)

Fig. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | BIOCHEMISTRY, vol. 18, no. 19, 18th September 1979, pages 4173-4176, American Chemical Societ, US; V. RHODEN et al.: "Formation of unilamellar lipid vesicles of controllable dimensions by detergent dialysis" * Abstract; page 4174, paragraph 2 * | 1-8 | A 61 K 9/50 |
| A | CHEMICAL ABSTRACTS, vol. 89, 1978, pages 171-172, abstract no. 1800k, Columbus, Ohio, US; S.M. GOLDIN et al.: "Reconstitution and "transport specificity fractionation" of the human erythrocyte glucose transport system. A new approach for identification and isolation of membrane transport proteins", & J. Biol. CHEM. 1978, 253(8), 2575-83 * Whole abstract * | 1-8 | |
| Y | JOURNAL OF CONTROLLED RELEASE, vol. 5, no. 3, 1988, pages 203-209, Elsevier Science Publishers B.V.; S.G. WOOLFREY et al.: "Pulmonary absorption of liposome-encapsulated 6-carboxyfluorescein" * Page 204: "Preparation of liposomes" * | 1-8 | TECHNICAL FIELDS SEARCHED (Int. Cl.4)<br><br>A 61 K |

The present search report has been drawn up for all claims

| Place of search | Date of completion of the search | Examiner |
|---|---|---|
| THE HAGUE | 16-05-1989 | BERTE M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................................
& : member of the same patent family, corresponding document

EPO FORM 1503 03.82 (P0401)